# NOUVEAU FASCICULE DE BREVET EUROPEEN

(11) **EP 0 984 772 B2**
(45) Date de publication et mention de la décision concernant l'opposition: **05.06.2013**
(45) Mention de la délivrance du brevet: 11.08.2004
(21) Numéro de dépôt: 99910448.2
(22) Date de dépôt: 29.03.1999
(51) Int. Cl.: A61K 9/14, C01B 33/193, A23K 1/16

(54) **COMPOSITION COMPRENANT UN LIQUIDE ABSORBE SUR UN SUPPORT A BASE DE SILICE PRECIPITEE**
ZUSAMMENSETZUNG BESTEHEND AUS EINER FLÜSSIGKEIT, DIE AUF EINEM TRÄGER AUS GEFÄLLTER KIESELSÄURE ABSORBIERT IST
COMPOSITION COMPRISING A LIQUID ABSORBED ON A SUPPORT BASED ON PRECIPITATE SILICA

(30) Priorité: 30.03.1998 FR 9803909
(43) Date de publication de la demande: 15.03.2000
(73) Titulaire: RHODIA CHIMIE, 92408 Courbevoie Cédex (FR)
(72) Inventeur: VIOT, Jean-François, F-69540 Irigny (FR)
(74) Mandataire: Delenne, Marc
(86) Numéro de dépôt international: PCT/FR1999/000723
(87) Numéro de publication internationale: WO 1999/049850

(56) Documents cités:
- EP-A- 0 345 109
- EP-A1- 0 937 755
- WO-A1-96/32949
- US- - 4 617 294
- US-A- 4 617 294
- US-A- 4 717 561
- US-A- 4 775 540
- US-A- 4 820 532
- PPG INDUSTRIES INC.: 'Hi-Sil SC60' HI-SIL SC60 BENEFITS Septembre 1996,
- BEWER PRODUKTCHARAKTERISIERUNG VERSCHIEDENER KIESELSÄUREN UND DER ENTSPRECHENDEN VIT.E-ADSORBATE (LABORPRODUKTE) 07 Février 1997, page 9
- DEGUSSA AG: 'Analysenzertifikat' 10 Mai 2005, page 10
- BEWERT, DR.: 'Analysenbericht' KIESELSÄUREN UND VITAMIN E-ADSORBATE (TRÄGER VON DEGUSSA) LABORPRODUKTE 27 Novembre 1996, page 1
- BASF AG: 'Dokumentation einer nicht beschriebenen Prüfung' 21 Novembre 1996, pages 1 - 3
- GRUSS, M.: 'Kontaktbericht' KONTAKTAUFBAU ZUM VITAMIN-MARKETING DER BASF FÜR DIE VITAMIN E 21 Novembre 1996, pages 15 - 16
- HEMMERLÉ, ET AL.: '"NF T 30-022" zur Bestimmung der Ölzahl' PEINTURES DÉTERMINATION DE LA PRISE D'HUILE DES PIGMENTS ET MATIÈRES DE CHARGE 31 Mars 1953,
- HARNISCH, R. ET AL.: '"Chemische Technologie", Band 3 (Anorganische Technologie II)', vol. 3.6, 1983, CARL HANSER VERLAG, MÜNCHEN-WIEN page 88
- SCOTT ET AL.: 'High capacity precipitated silica for the rubber industry' 148TH MEETING OF THE RUBBER DIVISION AMERICAN CHEMICAL SOCIETY no. 113, 1995, pages 8,12 - 14
- 'Daten aus Untersuchungsbefund UB 15001 zu Hi-Sil SC60' D2B, EINSPRUCHSVERFAHREN GEGEN EP 0 984772 B1 ANALYTIKBEFUND ZU HI-SIL SC 60-M 07 Janvier 1998, page 1
- PPG INDUSTRIES, INC.: 'Hi-Sil SC 72' HI-SIL SC72 BENEFITS Septembre 1996,
- 'Untersuchungsbefund UB 14998 zu Hi-Sil SC 72' D3A, EINSPRUCHSVERFAHREN GEGEN EP 0 984 772 B1 ANALYTIKBEFUND ZU HI-SIL SC 72 12 Décembre 1997,
- 'Daten aus Untersuchungsbefund UB 15002 zu Hi-Sil SC 72' D3B, EINSPRUCHSVERFAHREN GEGEN EP 0 984 772 B1 ANALYTIKBEFUND ZU HI-SIL SC 72 07 Janvier 1998,

## Description

La présente invention concerne une composition comprenant un complément liquide d'alimentation animale absorbé sur un support à base d'une silice précipitée particulière.

Il est connu de conditionner des liquides, notamment des additifs d'alimentation animale, sur des supports solides, en particulier sur un support silice. Ce conditionnement a généralement pour but de transformer un liquide non ou difficilement manipulable en poudre fluide pouvant être stockée facilement, par exemple en sac, et manipulable plus aisément, et pouvant aussi se disperser sans difficulté et bien se mélanger à d'autres constituants solides divisés.

Dans l'exposé qui suit, on entend par composition conditionnée la composition ainsi obtenue, c'est-à-dire un liquide absorbé sur un support silice.

Cette composition conditionnée doit pouvoir être manipulée facilement, ce qui implique une bonne fluidité et un faible poussiérage. Elle doit également présenter une teneur assez importante en matière active (liquide), ainsi qu'une densité assez élevée. Ces différentes exigences sont parfois contradictoires et ne sont pas souvent remplies par les supports silices de l'art antérieure (voir les brevets US-A-4 617 294, US-A-4 717 561, US-A-4 820 532 et EP-A-0 345 109).

Ainsi, l'objet principal de l'invention est de fournir une nouvelle présentation de composition conditionnée possédant de plus, de manière avantageuse, à la fois une bonne fluidité, un poussiérage faible voire nul et une densité préférentiellement assez élevée.

La Demanderesse a trouvé que, dans ce but, l'utilisation d'une silice précipitée ayant, entre autres, une morphologie bien spécifique, en l'occurrence une présentation sous forme de billes sensiblement sphériques, et une taille moyenne des particules relativement élevée, en tant que support pour compléments liquides d'alimentation animale, par exemple pour le chlorhydrate de choline, était particulièrement satisfaisante.

Dans l'exposé qui suit, la taille moyenne des particules est mesurée selon la norme NF X 11507 (décembre 1970) par tamisage à sec et détermination du diamètre correspondant à un refus cumulé de 50 %.

La densité de remplissage à l'état tassé (DRT) est déterminée selon la norme NF T 30-042.

La prise d'huile DOP est mesurée selon la norme NF T 30-022 (mars 1953) en mettant en oeuvre le dioctylphtalate.

Les volumes poreux donnés sont mesurés par porosimétrie au mercure : la préparation de chaque échantillon peut se faire comme suit : chaque échantillon est préalablement séché pendant 2 heures en étuve à 200 °C. puis placé dans un récipient à essai dans les 5 minutes suivant sa sortie de l'étuve et dégazé sous vide, par exemple à l'aide d'une pompe à tiroirs rotatifs ; les diamètres de pores sont calculés par la relation de WASHBURN avec un angle de contact théta égal à 140° et une tension superficielle gamma égale à 484 Dynes/cm (porosimètre MICROMERITICS 9300).

La surface spécifique BET est déterminée selon la méthode de BRUNAUER - EMMET - TELLER décrite dans "The journal of the Amencan Chemical Society". Vol. 60, page 309, février 1938 et correspondant à la norme NF T 45007 (novembre 1987).

La surface spécifique CTAB est la surface externe déterminée selon la norme NF T 45007 (novembre 1987) (5.12).

Le temps d'écoulement tₑ des compositions conditionnées, qui illustre leur fluidité, est mesuré par passage de 50 grammes de produit à travers un silo en verre d'orifice calibré : diamètre du cylindre : 50 mm ; hauteur du cylindre : 64 mm ; angle de cône 53 ° ; diamètre de passage à la base du cône 12 mm. Selon cette méthode, on remplit le silo, fermé à sa base, à l'aide de 50 grammes de produit ; puis on ouvre sa base et on note après écoulement total desdits 50 grammes le temps de passage, dit temps d'écoulement tₑ, du produit.

La composition selon l'invention comprend au moins un complément liquide d'alimentation animale absorbé sur un support contenant une silice précipitée, ladite silice précipitée se présentant sous forme de billes sensiblement sphériques et possédant :
- une taille moyenne des billes supérieure à 150 µm,
- une densité de remplissage à l'état tassé (DRT) comprise strictement entre 0,24 et 0,29,
- un taux de refus au tamis ayant une ouverture de mailles de 75 µm d'au moins 88 % en poids,
- un volume poreux (V_{d1}) constitué par les pores de diamètre inférieur à 1 µm d'au moins 2,1 cm³/g,
- une prise d'huile DOP d'au moins 275 ml/100g,
   ladite composition présentant une teneur en complément liquide d'alimentation animale d'au moins 60% en poids.

Ainsi, la silice précipitée utilisée dans la composition conditionnée selon l'invention se présente sous une forme très particulière, en l'occurrence sous forme de billes sensiblement sphériques.

La taille moyenne desdites billes est supérieure à 150 µm, et, avantageusement, égale à au moins 200 µm; en général, elle est d'au plus 320 µm, de préférence d'au plus 300 µm; elle peut être comprise entre 200 et 290 µm, en particulier entre 210 et 285 µm, par exemple entre 215 et 280 µm. Cette taille peut notamment être comprise entre 260 et 280 µm.

La densité de remplissage à l'état tassé (DRT) de cette silice précipitée est relativement haute et comprise strictement entre 0,24 et 0,29, en particulier comprise entre 0,25 et 0,28.

Cette silice présente un taux de refus au tamis ayant une ouverture de mailles de 75 µm d'au moins 88 % en poids. Ceci signifie qu'au moins 88 % en poids des particules de cette silice sont retenus par un tamis dont l'ouverture de mailles est de 75 µm.

Ainsi, cette silice présente une teneur en poids de particules fines plutôt faible.

De manière préférée, son taux de refus au tamis ayant une ouverture de mailles de 75 µm est d'au moins 90 % en poids, en particulier d'au moins 92 % en poids, voire d'au moins 93 % en poids, et il peut être inférieur à 94 % en poids.

Il est par exemple d'au moins 90 % en poids et inférieur à 96 % en poids, notamment à 94 % en poids.

La silice précipitée utilisée dans la composition conditionnée selon l'invention est donc assez peu poussiérante.

Ladite silice possède un volume poreux (V_{d1}) constitué par les pores de diamètre inférieur à 1 µm d'au moins 2,1 cm³/g, par exemple d'au moins 2,2 cm³/g.

Elle présente une pnse d'huile DOP d'au moins 275 ml/100g, en particulier supérieure à 280 ml/100g. Celle-ci peut être ainsi comprise entre 275 et 320 ml/100g, par exemple entre 280 et 310 ml/100g, notamment entre 280 et 295 ml/100g.

Sa surface spécifique BET est généralement comprise entre 140 et 240 m²/g, notamment entre 140 et 200 m²/g. Elle est par exemple comprise entre 150 et 190 m²/g. Elle peut être en particulier comprise entre 160 et 170 m²/g.

Sa surface spécifique CTAB peut être comprise entre 140 et 230 m²/g, notamment entre 140 et 190 m²/g. Elle est par exemple comprise entre 150 et 180 m²/g, en particulier entre 150 et 165 m²/g.

Elle présente en général une humidité réduite, son taux d'humidité (perte au séchage à 105 °C pendant 2 heures) est de préférence inférieur à 6 % en poids, par exemple inférieur à 5 % en poids.

De manière avantageuse, la silice employée dans la composition selon invention est issue du séchage au moyen d'un atomiseur à buses d'une suspension de silice obtenue par précipitation. De préférence, ladite suspension de silice à sécher présente un taux de matière sèche compris entre 18,0 et 20,5 % en poids, en particulier entre 18,5 et 20,0 % en poids, notamment entre 19,0 et 20,0 % en poids.

Cette silice peut être préparée par un procédé du type comprenant la réaction d'un silicate avec un agent acidifiant ce par quoi l'on obtient une suspension de silice précipitée, puis la séparation et le séchage à l'aide d'un atomiseur à buses de cette suspension, la précipitation étant réalisée de la manière suivante :
(1) on forme un pied de cuve initial comportant au moins une partie de la quantité totale de silicate engagé dans la réaction et, en général, au moins un électrolyte, la concentration en silicate (exprimée en SiO₂) dans ledit pied de cuve initial étant inférieure à 100 g/l, notamment à 90 g/l, et la concentration en électrolyte (sulfate de sodium par exemple) dans ledit pied de cuve initial étant inférieure à 17 g/l, par exemple inférieure à 14 g/l,
(2) on ajoute l'agent acidifiant audit pied de cuve jusqu'à l'obtention d'une valeur du pH du milieu réactionnel d'au moins environ 7, généralement comprise entre environ 7 et 8,
(3) on ajoute au milieu réactionnel de l'agent acifiant et, le cas échéant, simultanément la quantité restante du silicate,
la suspension à sécher présentant un taux de matière sèche compris entre 18,0 et 20,5 % en poids, en particulier entre 18,5 et 20,0 % en poids.

il est à noter, d'une manière générale, que le procédé concerné est un procédé de synthèse de silice de précipitation, c'est-à-dire que l'on fait agir, dans des conditions particulières, un agent acidifiant sur un silicate.

Le choix de l'agent acidifiant et du silicate se fait d'une manière bien connue en soi.

On peut rappeler que l'on utilise généralement comme agent acidifiant un acide minéral fort tel que l'acide sulfurique, l'acide nitrique ou l'acide chlorhydrique, ou un acide organique tel que l'acide acétique, l'acide formique ou l'acide carbonique.

L'agent acidifiant peut être dilué ou concentré ; sa normalité peut être comprise entre 0,4 et 8 N, par exemple entre 0,6 et 1,5 N.

En particulier, dans le cas où l'agent acidifiant est l'acide sulfurique, sa concentration peut être comprise entre 40 et 180 g/l, par exemple entre 60 et 130 g/l.

On peut par ailleurs utiliser en tant que silicate toute forme courante de silicates tels que métasilicates, disilicates et avantageusement un silicate de métal alcalin, notamment le silicate de sodium ou de potassium.

Le silicate peut présenter une concentration exprimée en silice comprise entre 40 et 330 g/l, en particulier entre 60 et 300 g/l, par exemple entre 60 et 250 g/l.

De manière générale, on emploie, comme agent acidifiant, l'acide sulfurique, et, comme silicate, le silicate de sodium.

Dans le cas où l'on utilise le silicate de sodium, celui-ci présente, en général, un rapport pondéral SiO₂/Na₂O compris entre 2 et 4, par exemple entre 3.0 et 3,7.

Le pied de cuve initial comprend en général un électrolyte. Le terme d'électrolyte s'entend ici dans son acceptation normale, c'est-à-dire qu'il signifie toute substance ionique ou moléculaire qui, lorsqu'elle est en solution, se décompose ou se dissocie pour former des ions ou des particules chargées. On peut citer comme électrolyte un sel du groupe des sels des métaux alcalins et alcalino-terreux, notamment le sel du métal de silicate de départ et de l'agent acidifiant, par exemple le chlorure de sodium dans le cas de la réaction d'un silicate de sodium avec l'acide chlorhydrique ou, de préférence, le sulfate de sodium dans le cas de la réaction d'un silicate de sodium avec l'acide sulfurique.

Dans le cas (préféré) d'un pied de cuve de départ ne comprenant qu'une partie de la quantité totale de silicate engagé dans la réaction, on procède, dans l'étape (3), à une addition simultanée d'agent acidifiant et de la quantité restante de silicate.

Cette addition simultanée est de préférence réalisée de manière telle que la valeur du pH soit constamment égale (à +/- 0,2 près) à celle atteinte à l'issue de l'étape (2).

En général, dans une étape suivante, on ajoute au milieu réactionnel une quantité supplémentaire d'agent acidifiant, de préférence jusqu'à l'obtention d'une valeur du pH du milieu réactionnel comprise entre 3 et 6,5, en particulier entre 4 et 6,5.

Il peut être alors avantageux d'effectuer, après cette addition d'une quantité supplémentaire d'agent acidifiant, un mûrissement du milieu réactionnel, ce mûrissement pouvant par exemple durer de 2 à 60 minutes, notamment de 3 à 20 minutes.

Dans le cas d'un pied de cuve de départ comprenant la quantité totale du silicate engagé dans la réaction, on procède, dans l'étape (3), à une addition d'agent acidifiant, de préférence jusqu'à l'obtention d'une valeur du pH du milieu réactionnel comprise entre 3 et 6,5, en particulier entre 4 et 6,5.

Il peut être également alors avantageux d'effectuer, après cette étape (3), un mûrissement du milieu réactionnel, ce mûrissement pouvant par exemple durer de 2 à 60 minutes, notamment de 3 à 20 minutes.

La température du milieu réactionnel est généralement comprise entre 70 et 98 °C.

Selon une variante du procédé, la réaction est effectuée à une température constante, de préférence comprise entre 80 et 95 °C.

Selon une autre variante (préférée) du procédé, la température de fin de réaction est plus élevée que la température de début de réaction ainsi, on maintient la température au début de la réaction de préférence entre 70 et 95 °C, puis on augmente la température, de préférence jusqu'à une valeur comprise entre 80 et 98 °C, valeur à laquelle elle est maintenue jusqu'à la fin de la réaction.

On obtient, à l'issue des étapes qui viennent d'être decrites, une bouillie de silice qui est ensuite séparée (séparation liquide-solide).

En général, ladite séparation comprend une filtration et un lavage à l'aide d'un filtre équipé d'un moyen de compactage.

Ce filtre peut être un filtre à bande équipé d'un rouleau assurant le compactage.

Néanmoins, de préférence, ce filtre est un filtre presse, la séparation comprend alors en général une filtration, un lavage puis un compactage, au moyen dudit filtre.

La suspension de silice précipitée ainsi récupérée (gâteau de filtration) est ensuite séchée par atomisation, au moyen d'un atomiseur à buses.

De manière très préférée, cette suspension doit présenter immédiatement avant son séchage un taux de matière sèche compris entre 18,0 et 20,5 % en poids, en particulier entre 18,5 et 20,0 % en poids, par exemple entre 19,0 et 20,0 % en poids.

Il y a lieu de noter que le gâteau de filtration n'est pas toujours dans des conditions permettant une atomisation notamment à cause de sa viscosité élevée. D'une manière connue en soi, on soumet alors le gâteau à une opération de délitage. Cette opération peut être réalisée par passage du gâteau dans un broyeur de type colloïdal ou à bille. Le délitage est généralement effectué en présence d'un composé de l'aluminium, en particulier d'aluminate de sodium. L'opération de délitage permet notamment d'abaisser la viscosité de la suspension à sécher ultérieurement.

La Demanderesse a découvert que la silice précipitée définie plus haut, donc ayant une morphologie bien spécifique, en l'occurrence une présentation sous forme de billes sensiblement sphériques, et denses, une taille moyenne des particules relativement élevée, présentait une bonne fluidité et un caractère peu poussiérant, et convenait particulièrement bien au conditionnement des compléments liquides d'alimentation animale.

La composition conditionnée conforme à l'invention contient un complément liquide d'alimentation animale. On peut notamment citer des vitamines, telles que la vitamine E, la choline et, de préférence, le chlorhydrate de choline.

L'opération d'absorption dudit liquide sur le support à base de ladite silice précipitée peut s'effectuer de manière classique, en particulier par pulvérisation dudit liquide sur la silice dans un mélangeur.

La composition selon l'invention présente notamment dans le cas du chlorhydrate de choline, une teneur en complément liquide d'alimentation animale d'au moins 60 % en poids, en particulier comprise entre 60 et 75 % en poids, notamment entre 60 et 70 % en poids (par rapport au poids total de la composition) ; elle peut être par exemple comprise entre 63 et 68 % en poids, notamment entre 64 et 67 % en poids.

La composition conditionnée selon l'invention, du fait de la présence de la silice précipitée ayant les caractéristiques sus-mentionnées, présente, de manière avantageuse, notamment dans le cas du chlorhydrate de choline et en particulier pour une teneur en chlorhydrate de choline comprise entre 63 et 68 % en poids, de préférence entre 64 et 67 % en poids, un poussiérage faible voire nul et une très bonne fluidité, combinés, en général, à une densité élevée.

La composition conditionnée selon l'invention, en particulier dans le cas du chlorhydrate de choline, présente, de manière préférée, un temps d'écoulement tₑ (mesuré pour 50 grammes de produit et pour un diamètre de passage de 12 mm) inférieur à 11 secondes, en particulier d'au plus 10 secondes, notamment inférieur à 7 secondes, ce qui témoigne de sa très bonne fluidité.

De plus, la composition conditionnée conforme à l'invention, en particulier dans le cas du chlorhydrate de choline, présente habituellement une densité de remplissage à l'état tassé (DRT) d'au moins 0,68, par exemple d'au moins 0,70, notamment d'au moins 0,72.

Enfin, en général, ladite silice précipitée confère à cette composition, en particulier dans le cas du chlorhydrate de choline, un taux de refus au tamis ayant une ouverture de mailles de 75 µm d'au moins 95 % en poids, notamment d'au moins 96 % en poids. Ce taux, en particulier dans le cas du chlorhydrate de choline, est préférentiellement d'au moins 97 % en poids, par exemple d'au moins 98 % en poids. Cette composition présente ainsi habituellement une teneur en poids de particules fines très faible. Elle est donc peu ou voire pas poussiérante.

L'exemple suivant illustre l'invention.

### EXEMPLE

1) Dans un réacteur en acier inoxydable muni d'un système d'agitation par hélices et d'un chauffage par double enveloppe, on introduit :
   - 345 litres d'eau
   - 7,5 kg de Na₂SO₄
   - 588 litres de silicate de sodium aqueux présentant un rapport pondéral SiO₂/Na₂O égal à 3,5 et une densité à 20 °C égale à 1,133.
   La concentration en silicate exprimée en SiO₂ dans le pied de cuve initial est ainsi de 85 g/l. Le mélange est alors porté à une température de 79 °C tout en le maintenant sous agitation. On y introduit ensuite 387 litres d'acide sulfurique dilué, de densité à 20°C égale à 1,050, jusqu'à obtenir dans le mileu réactionnel une valeur de pH (mesurée à sa température) égale à 8,0. La température de la réaction est de 79 °C pendant les 25 premières minutes ; elle est ensuite portée de 79 à 86 °C en 15 minutes, puis maintenue à 86 ° C jusqu'à la fin de la réaction.
   On introduit ensuite (c'est-à-dire lorsque le pH du milieu réactionnel a atteint la valeur de 8,0) conjointement dans le milieu réactionnel 82 litres de silicate de sodium aqueux du type décrit ci-avant et 134 litres d'acide sulfurique, également du type décrit ci-avant, cette introduction simultanée d'acide et de silicate étant réalisée de manière telle que le pH du milieu réactionnel, pendant la période d'introduction, soit constamment égal à 8,0 ± 0,1. Après introduction de la totalité du silicate, on continue à introduire de l'acide dilué pendant 9 minutes de manière à amener le pH du milieu réactionnel à une valeur égale à 5,2. Après cette introduction d'acide, on maintient la bouillie réactionnelle obtenue pendant 5 minutes sous agitation.
   La durée totale de la réaction est de 118 minutes.
   On obtient ainsi une bouillie ou suspension de silice précipitée qui est ensuite filtrée et lavée au moyen d'un filtre presse à plateaux verticaux (lesdits plateaux étant équipés de membrane déformable permettant de comprimer le gâteau de filtration par introduction d'air sous pression), à une pression de 4,5 bars et pendant le temps nécessaire afin d'obtenir un gâteau de silice dont la perte au feu est égale à 80,5 % (donc un taux de matière sèche de 19,5 % en poids).
   Le gâteau obtenu est ensuite fluidifié par action mécanique et chimique (ajout d'une quantité d'aluminate de sodium correspondant à un rapport pondéral Al/SiO₂ de 3000 ppm); pendant cette opération, on ajoute de l'eau de manière à obtenir une bouillie ayant une perte au feu égale à 81.5 % (donc un taux de matière sèche de 18,5 % en poids). Après cette opération de délitage, la bouillie résultante, de pH égal à 6,4, est séchée au moyen d'un atomiseur à buses.
   La silice précipitée obtenue P1 se présente sous forme de billes sensiblement sphériques et possède les caractéristiques supplémentaires suivantes :
   - taille moyenne des particules 260 µm
   - DRT 0,28
   - taux de refus au tamis 75 µm 93,0 %
   - prise d'huile DOP 290 ml/100g
   - volume poreux (V_{d1}) constitué par les pores de d < 1 µm 2,1 cm³/g
2) On met du chlorhydrate de choline sur un support formé par la silice P1 préparée en 1).
   La mise sur support du chlorhydrate de choline s'effectue dans un mélangeur en V de 7 litres tournant à 20 tours/min, avec un axe intérieur tournant à 1900 tours/min, muni de plaques au travers desquelles est pulvérisé le chlorhydrate de choline et sur lesquelles sont fixés des couteaux émotteurs.
   On charge la totalité de la silice P1 dans le mélangeur, puis on pulvérise le chlorhydrate de choline (à une température de 70 °C et à un débit de 100 m)/min) sur cette silice. On mélange pendant 15 minutes, puis on homogénéise durant 2 minutes supplémentaires.
   La composition conditionnée alors obtenue contient 34 % en poids de silice précipitée P1 et 66 % en poids de chlorhydrate de choline et possède les caractéristiques supplémentaires suivantes :
   - temps d'écoulement tₑ 6,5 secondes
   - DRT 0,70
   - taux de refus au tamis 75 µm > 95 %

Ainsi, cette composition conditionnée à base d'un support silice précipitée, sous forme de billes sensiblement sphénques, présente une très bonne fluidité (ce qui est illustré notamment par un faible temps d'écoulement tₑ) et n'est pas poussiérante (ce qui est notamment illustré par un taux de refus au tamis ayant une ouverture de mailles de 75 µm élevé), tout en ayant une densité plutôt élevée.

## Revendications

1. Composition conditionnée comprenant au moins un complément liquide d'alimentation animale absorbé sur un support contenant une silice précipitée, **caractérisée en ce que** ladite silice se présente sous forme de billes sensiblement sphénques et possède :
- une taille moyenne des billes supérieure à 150 µm,
- une densité de remplissage à l'état tassé (DRT) comprise strictement entre 0,24 et 0,29,
- un taux de refus au tamis ayant une ouverture de mailles de 75 µm d'au moins 88 % en poids,
- un volume poreux (V_{d1}) constitué par les pores de diamètre inférieur à 1 µm d'au moins 2,1 cm³/g.
- une prise d'huile DOP d'au moins 27,5 ml/100g,
ladite composition présentant une teneur en complément liquide d'alimentation animale d'au moins 60% en poids.

2. Composition selon la revendication 1, **caractérisée en ce que** la dite silice possède une taille moyenne des billes d'au moins 200 µm, en particulier comprise entre 200 et 290 µm.

3. Composition selon l'une des revendications 1 et 2, **caractérisée en ce que** ladite silice possède un taux de refus au tamis ayant une ouverture de mailles de 75 µm d'au moins 90 % en poids.

4. Composition selon l'une des revendications 1 à 3, **caractérisée en ce que** ladite silice possède une prise d'huile DOP comprise entre 275 et 320 ml/100g.

5. Composition selon l'une des revendications 1 à 4, **caractérisée en ce que** ladite silice est issue du séchage au moyen d'un atomiseur à buses d'une suspension de silice obtenue par précipitation.

6. Composition selon la revendication 5, **caractérisée en ce que** ladite suspension de silice présente, avant séchage, un taux de matière sèche compris entre 18,0 et 20,5 % en poids, de préférence entre 18.5 et 20,0 % en poids.

7. Composition selon l'une des revendications 1 à 6, **caractérisée en ce que** ladite composition présente une teneur en complément liquide d'alimentation animale comprise entre 60 et 75 % en poids.

8. Composition selon l'une des revendications 1 à 7, **caractérisée en ce que** ledit complément liquide d'alimentation animale est le chlorhydrate de choline.

9. Composition selon l'une des revendications 1 à 8, **caractérisée en ce que** ladite composition présente un temps d'écoulement tₑ, pour 50 grammes et pour un diamètre de passage de 12 mm, inférieur à 11 secondes, de préférence d'au plus 10 secondes.

10. Composition selon la revendication 9, **caractérisée en ce que** ledit temps d'écoulement tₑ est inférieur à 7 secondes.

## Patentansprüche

1. Konditionierte Zusammensetzung umfassend wenigstens ein flüssiges Nahrungsergänzungsmittel für Tiere, das auf einem Träger absorbiert ist, der eine ausgefällte Silica enthält, **dadurch gekennzeichnet, dass** die Silica in Form von im Wesentlichen kugelförmigen Kugeln vorliegt und folgendes besitzt:
- eine mittlere Kugelgröße von größer als 150 µm,
- eine Fülldichte in dichtgedrängtem Zustand (DRT), die unbedingt zwischen 0,24 und 0,29 liegt,
- eine Rückstandsrate von wenigstens 88 Gew.-% in einem Sieb mit einer Maschenweite von 75 µm,
- ein Porenvolumen (V_{d1}) von wenigstens 2,1 cm³/g, das durch Poren mit einem Durchmesser von weniger als 1 µm gebildet ist,
- eine Ölzahl DOP von wenigstens 275 ml/100 g,
wobei die Zusammensetzung einen Gehalt des flüssigen Tiernahrungsergänzungsmittels von wenigstens 60 Gew.-% aufweist.

2. Zusammensetzung nach Anspruch 1, **dadurch gekennzeichnet, dass** die Silica eine mittlere Kugelgröße von wenigstens 200 µm besitzt, insbesondere zwischen 200 und 290 µm.

3. Zusammensetzung nach einem der Ansprüche 1 oder 2, **dadurch gekennzeichnet, dass** die Silica in einem Sieb mit einer Maschenweite von 75 µm eine Rückstandsrate von wenigstens 90 Gew.-% besitzt.

4. Zusammensetzung nach einem der Ansprüche 1 bis 3, **dadurch gekennzeichnet, dass** die Silica eine Ölzahl DOP zwischen 275 und 320 ml/100 g besitzt.

5. Zusammensetzung nach einem der Ansprüche 1 bis 4, **dadurch gekennzeichnet, dass** die Silica aus der Trocknung einer durch Fällung erhaltenen Silica-Suspension mittels eines Düsenzerstäubers stammt.

6. Zusammensetzung nach Anspruch 5, **dadurch gekennzeichnet, dass** die Silica-Suspension vor der Trocknung einen Trockensubstanzgehalt zwischen 18,0 und 20,5 Gew.-%, bevorzugt zwischen 18,5 und 20,0 Gew.-%, besitzt.

7. Zusammensetzung nach einem der Ansprüche 1 bis 6, **dadurch gekennzeichnet, dass** die Zusammensetzung einen Gehalt des flüssigen Tiernahrungsergänzungsmittels zwischen 60 und 75 Gew.-% aufweist.

8. Zusammensetzung nach einem der Ansprüche 1 bis 7, **dadurch gekennzeichnet, dass** das flüssige Tiernahrungsergänzungsmittel Cholinhydrochlorid ist.

9. Zusammensetzung nach einem der Ansprüche 1 bis 8, **dadurch gekennzeichnet, dass** die Zusammensetzung für 50 g und einen Durchgangsdurchmesser von 12 mm eine Auslaufzeit t_{θ} von weniger als 11 Sekunden, bevorzugt höchstens 10 Sekunden besitzt.

10. Zusammensetzung nach Anspruch 9, **dadurch gekennzeichnet, dass** die Auslaufzeit t_{θ} weniger als 7 Sekunden beträgt.

## Claims

1. Conditioned composition comprising at least one liquid supplement for animal feed absorbed on a support containing a precipitated silica, **characterized in that** the said silica is in the form of substantially spherical beads and has:
- an average bead size in excess of 150 µm,
- a packed filling density (PFD) of strictly between 0.24 and 0.29,
- a 75 µm screen oversize of at least 88% by weight,
- a pore volume (V_{d1}), made up of pores having a diameter smaller than 1 µm, of at least 2.1 cm³/g,
- a DOP oil absorption value of at least 275 ml/100 g,
said composition having a liquid supplement for animal feed content of at least 60% by weight.

2. Composition according to Claim 1, **characterized in that** the said silica has an average bead size of at least 200 µm, in particular between 200 and 290 µm.

3. Composition according to one of Claims 1 and 2, **characterized in that** the said silica has a 75 µm screen oversize of at least 90% by weight.

4. Composition according to one of Claims 1 to 3, **characterized in that** the said silica has a DOP oil absorption value between 275 and 320 ml/100 g.

5. Composition according to one of Claims 1 to 4, **characterized in that** the said silica results from the use of a nozzle atomizer to dry a suspension of silica obtained by precipitation.

6. Composition according to Claim 5, **characterized in that**, before it is dried, the said silica suspension has a solids content of between 18.0 and 20.5% by weight, preferably between 18.5 and 20.0% by weight.

7. Composition according to one of Claims 1 to 6, **characterized in that** the said composition has a liquid supplement for animal feed content between 60 and 75% by weight.

8. Composition according to one of Claims 1 to 7, **characterized in that** the said liquid supplement for animal feed is the choline hydrochloride.

9. Composition according to one of Claims 1 to 8, **characterized in that** the said composition has a flow time t_{f}, for 50 grams and for a passage diameter of 12 mm, of less than 11 seconds, preferably of at most 10 seconds.

10. Composition according to Claim 9, **characterized in that** the said flow time t_{f} is less than 7 seconds.
